# EUROPÄISCHE PATENTSCHRIFT

(11) **EP 0 723 551 B1**
(45) Veröffentlichungstag und Bekanntmachung des Hinweises auf die Patenterteilung: **06.03.2002**
(21) Anmeldenummer: 95928452.2
(22) Anmeldetag: 24.07.1995
(51) Int. Cl.: C07K 1/00, C07K 1/04, C07K 1/107, C07K 14/16, C07K 14/18, G01N 33/531, G01N 33/74, C07K 1/13

(54) **HAPTEN-MARKIERTE PEPTIDE**
HAPTEN-MARKED PEPTIDES
PEPTIDES MARQUES PAR UN HAPTENE

(30) Priorität: 25.07.1994 DE 4426276; 31.08.1994 DE 4430973
(43) Veröffentlichungstag der Anmeldung: 31.07.1996
(73) Patentinhaber: Roche Diagnostics GmbH, 68305 Mannheim (DE)
(72) Erfinder: HÖSS, Eva, D-82319 Starnberg (DE); SEIDEL, Christoph, D-82362 Weilheim (DE); WIENHUES, Ursula-Henrike, D-82152 Krailling (DE); FAATZ, Elke, D-82396 Pähl (DE); SCHMITT, Urban, D-82386 Oberhausen (DE)
(74) Vertreter: Weiss, Wolfgang, Dipl.-Chem. Dr.
(86) Internationale Anmeldenummer: EP9502921
(87) Internationale Veröffentlichungsnummer: WO9603423

(56) Entgegenhaltungen:
- EP-A- 0 117 648
- EP-A- 0 218 347
- WO-A-93/18054
- DE-A- 4 402 756
- US-A- 5 087 561

### SEQUENZPROTOKOLL

(1) ALLGEMEINE INFORMATION:
   (i) ANMELDER:
      (A) NAME: Boehringer Mannheim GmbH
      (B) STRASSE: Sandhofer Str. 116
      (C) ORT: Mannheim
      (E) LAND: Deutschland
      (F) POSTLEITZAHL: 68305
   (ii) ANMELDETITEL: Hapten-markierte Peptide
   (iii) ANZAHL DER SEQUENZEN: 17
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 17 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Human immunodeficiency virus type 1
   (viii) POSITION IM GENOM:
      (A) CHROMOSOM/SEGMENT: gp120
   (xi) SEQUENZBESCHREIBUNG:SEQ ID NO: 1:
   Asn Asn Thr Arg Lys Ser Ile Ser Ile Gly Pro Gly Arg Ala Phe Tyr
   1               5                   10                  15
   Thr
(2) INFORMATION ZU SEQ ID NO: 2:
   (i) SEQUENZ CHARAKTERISTIKA:
      (A) LÄNGE: 16 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Human immunodeficiency virus type 1
   (viii) POSITION IM GENOM:
      (A) CHROMOSOM/SEGMENT:gp120
   (xi) SEQUENZBESCHREIBUNG: SEQ ID NO: 2:
   Asn Thr Thr Arg Ser Ile Ser Ile Gly Pro Gly Arg Ala Phe Tyr Thr
   1               5                   10                  15
(2) INFORMATION ZU SEQ ID NO: 3:
   (i) SEQUENZ CHARAKTERISTIKA:
      (A) LÄNGE: 19 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Human immunodeficiency virus type 1
      (B) STAMM: Subtype O
   (viii) POSITION IM GENOM:
      (A) CHROMOSOM/SEGMENT: gp120
   (xi) SEQUENZBESCHREIBUNG: SEQ ID NO: 3:
   Ile Asp Ile Gln Glu Glu Arg Arg Met Arg Ile Gly Pro Gly Met Ala
   1               5                   10                  15
   Trp Tyr Ser
(2) INFORMATION ZU SEQ ID NO: 4:
   (i) SEQUENZ CHARAKTERISTIKA:
      (A) LÄNGE: 24 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Human immunodeficiency virus type 1
   (viii) POSITION IM GENOM:
      (A) CHROMOSOM/SEGMENT: gp41
   (xi) SEQUENZBESCHREIBUNG: SEQ ID NO: 4:
   Gln Ala Arg Ile Leu Ala Val Glu Arg Tyr Leu Lys Asp Gln Gln Leu
   1               5                   10                  15
   Leu Gly Ile Trp Gly Ala Ser Gly
               20
(2) INFORMATION ZU SEQ ID NO: 5:
   (i) SEQUENZ CHARAKTERISTIKA:
      (A) LÄNGE: 23 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Human immunodeficiency virus type 1
   (viii) POSITION IM GENOM:
      (A) CHROMOSOM/SEGMENT: gp41
   (xi) SEQUENZBESCHREIBUNG: SEQ ID NO: 5:
   Leu Gly Ile Trp Gly Cys Ser Gly Lys Leu Ile Cys Thr Thr Ala Val
   1               5                   10                  15
   Pro Trp Asn Ala Ser Trp Ser
               20
(2) INFORMATION ZU SEQ ID NO: 6:
   (i) SEQUENZ CHARAKTERISTIKA:
      (A) LÄNGE: 15 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Human immunodeficiency virus type 1
   (viii) POSITION IM GENOM:
      (A) CHROMOSOM/SEGMENT: gp41
   (xi) SEQUENZBESCHREIBUNG: SEQ ID NO: 6:
   Lys Asp Gln Gln Leu Leu Gly Ile Trp Gly Ser Ser Gly Lys Leu
   1               5                   10                  15
(2) INFORMATION ZU SEQ ID NO: 7:
   (i) SEQUENZ CHARAKTERISTIKA:
      (A) LÄNGE: 15 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Human immunodeficiencyvirus type 1
      (B) STAMM: Subtype O
   (viii) POSITION IM GENOM:
      (A) CHROMOSOM/SEGMENT: gp41
   (xi) SEQUENZBESCHREIBUNG: SEQ ID NO: 7:
   Ala Leu Glu Thr Leu Leu Gln Asn Gln Gln Leu Leu Ser Leu Trp
   1               5                   10                  15
(2) INFORMATION ZU SEQ ID NO: 8:
   (i) SEQUENZ CHARAKTERISTIKA:
      (A) LÄNGE: 15 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Human immunodeficiency virus type 1
      (B) STAMM: Subtype O
   (viii) POSITION IM GENOM:
      (A) CHROMOSOM/SEGMENT: gp41
   (xi) SEQUENZBESCHREIBUNG:SEQ ID NO: 8:
   Leu Ser Leu Trp Gly Cys Lys Gly Lys Leu Val Cys Tyr Thr Ser
   1               5                   10                  15
(2) INFORMATION ZU SEQ ID NO: 9:
   (i) SEQUENZ CHARAKTERISTIKA:
      (A) LÄNGE: 19 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Human immunodeficiency virus type 1
      (B) STAMM: Subtype O
   (viii) POSITION IM GENOM:
      (A) CHROMOSOM/SEGMENT: gp41
   (xi) SEQUENZBESCHREIBUNG: SEQ ID NO: 9:
   Trp Gly Ile Arg Gln Leu Arg Ala Arg Leu Leu Ala Leu Glu Thr Leu
   1               5                   10                  15
   Leu Gln Asn
(2) INFORMATION ZU SEQ ID NO: 10:
   (i) SEQUENZ CHARAKTERISTIKA:
      (A) LÄNGE: 27 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Human immunodeficiencyvirus type 2
   (viii) POSITION IM GENOM:
      (A) CHROMOSOM/SEGMENT: gp32
   (xi) SEQUENZBESCHREIBUNG:SEQ ID NO: 10:
   Gln Ala Gln Leu Asn Ser Trp Gly Cys Ala Phe Arg Gln Val Cys His
   1 5 10 15
   Thr Thr Val Pro Trp Pro Asn Asp Ser Leu Thr
   20 25
(2) INFORMATION ZU SEQ ID NO: 11:
   (i) SEQUENZ CHARAKTERISTIKA:
      (A) LÄNGE: 15 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Hepatitis C virus
   (viii) POSITION IM GENOM:
      (A) CHROMOSOM/SEGMENT: NS5
   (xi) SEQUENZBESCHREIBUNG:SEQ ID NO: 11:
   Ser Arg Arg Phe Ala Gln Ala Leu Pro Val Trp Ala Arg Pro Asp
   1 5 10 15
(2) INFORMATION ZU SEQ ID NO: 12:
   (i) SEQUENZ CHARAKTERISTIKA:
      (A) LÄNGE: 16 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Hepatitis C virus
   (viii) POSITION IM GENOM:
      (A) CHROMOSOM/SEGMENT: Core
   (xi) SEQUENZBESCHREIBUNG: SEQ ID NO: 12:
   Pro Gln Asp Val Lys Phe Pro Gly Gly Gly Gln Ile Val Gly Gly Val
   1 5 10 15
(2) INFORMATION ZU SEQ ID NO: 13:
   (i) SEQUENZ CHARAKTERISTIKA:
      (A) LÄNGE: 12 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Hepatitis C virus
   (viii) POSITION IM GENOM:
      (A) CHROMOSOM/SEGMENT: NS4
   (xi) SEQUENZBESCHREIBUNG: SEQ ID NO: 13:
   Glu Glu Ala Ser Gln His Leu Pro Tyr Ile Glu Gln
   1 5 10
(2) INFORMATION ZU SEQ ID NO: 14:
   (i) SEQUENZ CHARAKTERISTIKA:
      (A) LÄNGE: 9 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Hepatitis C Virus
   (viii) POSITION IM GENOM:
      (A) CHROMOSOM/SEGMENT: NS4
   (xi) SEQUENZBESCHREIBUNG:SEQ ID NO: 14:
   Gln Lys Ala Leu Gly Leu Leu Gln Thr
   1 5
(2) INFORMATION ZU SEQ ID NO: 15:
   (i) SEQUENZ CHARAKTERISTIKA:
      (A) LÄNGE: 18 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Hepatitis C Virus
   (viii) POSITION IM GENOM:
      (A) CHROMOSOM/SEGMENT: NS4
   (xi) SEQUENZBESCHREIBUNG: SEQ ID NO: 15:
   Ser Arg Gly Asn His Val Ser Pro Thr His Tyr Val Pro Glu Ser Asp
   1               5                   10                  15
   Ala Ala
(2) INFORMATION ZU SEQ ID NO: 16:
   (i) SEQUENZ CHARAKTERISTIKA:
      (A) LÄNGE: 28 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Hepatitis C Virus
   (viii) POSITION IM GENOM:
      (A) CHROMOSOM/SEGMENT: Core
   (xi) SEQUENZBESCHREIBUNG: SEQ ID NO: 16:
   Pro Gln Arg Lys Asn Lys Arg Asn Thr Asn Arg Arg Pro Gln Asp Val
   1               5                   10                  15
   Lys Phe Pro Gly Gly Gly Gln Ile Val Gly Val Val
               20                  25
(2) INFORMATION ZU SEQ ID NO: 17:
   (i) SEQUENZ CHARAKTERISTIKA:
      (A) LÄNGE: 25 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Hepatitis C Virus
   (viii) POSITION IM GENOM:
      (A) CHROMOSOM/SEGMENT:NS3
   (xi) SEQUENZBESCHREIBUNG:SEQ ID NO: 17:
   Ala Trp Tyr Glu Leu Thr Pro Ala Glu Thr Thr Val Arg Leu Arg Ala
       1               5                   10                  15
   Tyr Met Asn Thr Pro Gly Leu Pro Val
             20                  25

## Beschreibung

Die vorliegende Erfindung betrifft ein Verfahren zur Herstellung von Hapten-markierten Peptiden, durch dieses Verfahren erhältliche Hapten-markierte Peptide sowie die Verwendung dieser Peptide in einem immunologischen Nachweisverfahren.

Der Nachweis von Immunglobulinen in Körperflüssigkeiten, insbesondere in Humanseren, wird zur Diagnostik von Infektionen mit Mikroorganismen, insbesondere Viren, wie etwa HIV, Hepatitis-Viren, etc. verwendet. Das Vorhandensein von spezifischen Immunglobulinen in der untersuchten Probe wird üblicherweise durch Reaktion mit einem oder mehreren Antigenen, die mit den spezifischen Immunglobulinen reagieren, nachgewiesen. Verfahren zur Bestimmung von spezifischen Immunglobulinen in der Probeflüssigkeit müssen sensitiv, zuverlässig, einfach und schnell sein.

EP-A-117 648 offenbart in den Ansprüchen Konjugate mit einem Hapten, wobei das Hapten wenigstens eine antigene Determinante besitzt. In den letzten Jahren wurden zunehmend Nachweissysteme auf Basis nicht-radioaktiver Markierungsgruppen entwickelt, bei denen das Vorhandensein eines Analyten, z.B. eines spezifischen Antikörpers, in der untersuchten Probe mit Hilfe optischer (z.B. Lumineszenz oder Fluoreszenz), NMR-aktiver oder Metallpräzipitierender Detektionssysteme bestimmt werden konnte.

EP-A-0 307 149 offenbart einen Immuntest für einen Antikörper, bei dem zwei rekombinante Polypeptide als Antigene verwendet werden, von denen eines an einer festen Phase immobilisiert ist, und das andere eine Markierungsgruppe trägt, wobei beide rekombinanten Antigene in unterschiedlichen Organismen exprimiert werden, um die Spezifität des Nachweises zu erhöhen.

EP-A-0 366 673 offenbart ein Verfahren zum Nachweis von Antikörpern in einer Probe, bei dem ein Antikörper durch Reaktion mit einem gereinigten, markierten Antigen und dem gleichen gereinigten Antigen in einer Festphasen-gebundenen Form nachgewiesen wird. Als Antigen wird beispielsweise humanes IgG offenbart.

EP-A-0 386 713 beschreibt ein Verfahren zum Nachweis von Antikörpern gegen HIV unter Verwendung von zwei festen Trägern, wobei an beide festen Träger verschiedene HIV-Antigene immobilisiert werden, die jeweils mit einem Aliquot einer Probe und einem markierten HIV-Antigen in Kontakt gebracht werden, wobei das Vorhandensein von Antikörpern durch eine positive Reaktion in mindestens einem der Tests nachgewiesen wird. Als HIV-Antigene werden rekombinant hergestellte Polypeptide offenbart.

EP-A-0 507 586 beschreibt ein Verfahren zur Durchführung eines immunologischen Tests für ein spezifisches Immunglobulin, bei dem eine Probe mit zwei zur Bindung des Immunglobulins fähigen Antigenen in Kontakt gebracht wird, wobei das erste Antigen eine zur Bindung an einen festen Träger geeignete Gruppe trägt, und das zweite, Antigen eine Markierungsgruppe trägt. Die Markierungsgruppe kann eine direkte Markierungsgruppe sein, z.B. ein Enzym, ein Chromogen, ein Metallteilchen, oder auch eine indirekte Markierungsgruppe, d.h. die am Antigen angebrachte Markierungsgruppe kann mit einem Rezeptor für die Markierungsgruppe, der wiederum eine signalerzeugende Gruppe trägt, reagieren. Als Beispiel für eine solche indirekte Markierungsgruppe wird ein Fluoresceinderivat genannt, dessen Rezeptor ein Antikörper ist, der wiederum mit einem Enzym gekoppelt ist. Als Antigene werden Polypeptide, wie etwa das Hepatitis B-Oberflächenantigen offenbart. In dieses Antigen werden durch Derivatisierung SH-Gruppen eingeführt, mit denen das Fluorescein gekoppelt wird.

EP-A-0 507 587 offenbart ein spezifisch zum Nachweis von IgM Antikörpern geeignetes Verfahren, bei dem die Probe mit einem markierten Antigen, das gegen den nachzuweisenden Antikörper gerichtet ist, und einem zweiten Antikörper, der ebenfalls gegen den nachzuweisenden Antikörper gerichtet und an eine Festphase bindefähig ist, inkubiert wird.

Bei den aus dem Stand der Technik bekannten immunologischen Nachweisverfahren für Antikörper werden üblicherweise Polypeptid-Antigene verwendet, die meist durch rekombinante DNA-Methoden erzeugt wurden. Beim Einsatz derartiger PolypeptidAntigene können jedoch Probleme auftreten. So können rekombinante Polypetide oft nur in Form von Fusionspolypeptiden erzeugt werden, bei denen der Fusionsanteil zu falsch positiven Resultaten im Test führen kann. Weiterhin zeigen durch rekombinante Expression erzeugte Polypeptide oft eine nur geringe Stabilität in der Probelösung und neigen zu Aggregation. Ein weiterer Nachteil ist, daß oft keine selektive und reproduzierbare Einführung von Markierungsgruppen in solche Polypeptide möglich ist.

Überdies ist die Herstellung rekombinanter Polypeptidantigene mit hohen Kosten verbunden und es können große Schwankungen in der immunologischen Reaktivität bei verschiedenen Chargen rekombinanter Polypeptidantigene auftreten.

Das der vorliegenden Erfindung zugrundeliegende Problem war somit die Bereitstellung eines Verfahrens, bei dem auf einfache und effiziente Weise Antigene für immunologische Tests erzeugt werden können, wobei die Nachteile der aus dem Stand der Technik bekannten Antigene mindestens teilweise beseitigt werden. Weiterhin soll das Verfahren eine selektive und reproduzierbare Einführung von Markierungsgruppen in die Antigene ermöglichen.

Dieses Problem wird gelöst durch ein Verfahren zur Herstellung von Hapten-markierten Peptiden, welches dadurch gekennzeichnet ist,
daß man
(a) ein Peptid mit der gewünschten Aminosäuresequenz an einer Festphase aus Aminosäurederivaten synthetisiert, deren reaktive Seitengruppen durch Schutzgruppen blockiert sind, wobei die Schutzgruppen an primären Aminoseitengruppen derart ausgewählt werden, daß sie gegebenenfalls selektiv abspaltbar sind,
(b) eine Abspaltung von Schutzgruppen durchführt, wobei mindestens eine freie primäre Aminogruppe entsteht,
(c) ein Hapten-Aktivester-derivat an die mindestens eine freie primäre Aminogruppe des Peptids koppelt, und
(d) gegebenenfalls eine Abspaltung von noch verbleibenden Schutzgruppen durchführt, wobei das Hapten ausgewählt wird aus der Gruppe bestehend aus Sterinen, Gallensäuren, Sexualhormonen, Corticoiden, Cardenoliden, Cardenolid-Glycosiden, Bufadienoliden, Steroid-Sapogeninen und Steroidalkaloiden.

Die durch das erfindungsgemäße Verfahren hergestellten Peptide haben vorzugsweise eine Länge von maximal 50 Aminosäuren, besonders bevorzugt von maximal 30 Aminosäuren und eignen sich hervorragend für immunologische Nachweisverfahren, insbesondere zur Bestimmung von spezifischen Immunglobulinen. Überraschenderweise wurde festgestellt, daß die durch das erfindungsgemäße Verfahren hergestellten Peptide trotz der Anwesenheit von sperrigen Hapten-Markierungsgruppen eine hohe Affinität und Spezifität für die nachzuweisenden Immunglobuline besitzen.

Das erfindungsgemäße Verfahren ermöglicht die selektive Einführung von Hapten-Markierungsgruppen, sowohl bezüglich ihrer Lokalisierung als auch ihrer Anzahl. Bei der erfindungsgemäßen Peptidsynthese besteht nämlich die Möglichkeit, durch Verwendung bestimmter Schutzgruppen an primären Aminogruppen der eingesetzten Aminosäurederivate diejenigen Positionen des Peptids gezielt auszuwählen, die nach selektiver Schutzgruppenabspaltung zur Reaktion mit dem Hapten zur Verfügung stehen. Auf diese Weise wird eine bessere Reproduzierbarkeit und Sensitivität der Tests erreicht.

Ein weiterer Vorteil des erfindungsgemäßen Verfahrens ist, daß durch die Verwendung von Peptid-Antigenen alle Antikörperklassen, wie etwa IgG, IgM, IgE und IgA, erkannt werden. Auch die Störanfälligkeit des Tests ist durch Verwendung definierter kleiner und stabiler Antigene, die nicht zur Aggregation neigen, geringer.

Die Haptene, die durch das erfindungsgemäße Verfahren an das Peptid gekoppelt werden, sind Moleküle mit einem Steroidgrundgerüst, die ausgewählt werden aus der Gruppe bestehend aus Sterinen, Gallensäuren, Sexualhormonen, Corticoiden, Cardenoliden, Cardenolid-Glycosiden, Bufadienoliden, Steroid-Sapogeninen und Steroidalkaloiden. Diese Haptene sind mit einem spezifischen Rezeptor bindefähig, z.B. mit Antikörpern oder Antikörperfragmenten, die gegen das Hapten gerichtet sind. Besonders bevorzugt wird das Hapten ausgewählt aus der Gruppe bestehend aus Cardenoliden und Cardenolid-Glycosiden. Vertreter dieser Stoffklassen sind Digoxigenin, Digitoxigenin, Gitoxigenin, Strophantidin, Digoxin, Digitoxin, Ditoxin und Strophantin, wobei Digoxigenin und Digoxin besonders bevorzugt sind.

Bei dem erfindungsgemäßen Verfahren wird das Hapten-Aktivester-derivat an den Aminoterminus oder/und an freie primäre Aminoseitengruppen des Peptids gekoppelt. Der Begriff "Aktivester" im Sinne der vorliegenden Erfindung umfaßt aktivierte Estergruppen, die mit freien Aminogruppen von Peptiden unter solchen Bedingungen reagieren können, daß keine störenden Nebenreaktionen mit anderen reaktiven Gruppen des Peptids auftreten können. Vorzugsweise wird als Aktivesterderivat ein N-Hydroxysuccinimidester verwendet. Beispiele für geeignete Hapten-Aktivesterderivate sind Digoxin-4'''-hemiglutarat-N-hydroxysuccinimidester, Digoxigenin-3-carboxymethylether-N-hydroxysuccinimidester, Digoxigenin-3-O-methylcarbonyl-∈-aminocapronsäure-N-hydroxysuccinimidester, Digoxigenin-3-hemisuccinat-N-hydroxysuccinimidester, Digitoxin-4'''-hemiglutarat-N-hydroxysuccinimidester und Digitoxigenin-3-hemisuccinat-N-hydroxysuccinimidester. Diese Haptenderivate sind von der Fa. Boehringer Mannheim GmbH (Mannheim, BRD) kommerziell erhältlich. Neben den N-Hydroxysuccinimidestern können auch analoge p-Nitrophenyl-, Pentafluorphenyl-, Imidazolyl- oder N-Hydroxybenzotriazolylester verwendet werden.

Bei dem erfindungsgemäßen Verfahren wird das Peptid mit der gewünschten Aminosäuresequenz an einer Festphase, vorzugsweise mit einem kommerziellen Peptid-Synthesegerät (z.B. die Geräte A 431 oder A 433 von Applied Biosystems) hergestellt. Die Synthese erfolgt nach bekannten Methoden, vorzugsweise ausgehend vom Carboxylterminus des Peptids unter Verwendung von Aminosäurederivaten. Vorzugsweise werden Aminosäurederivate eingesetzt, deren für die Kupplung benötigte Amino-Endgruppe mit einem Fluorenylmethyloxycarbonyl (Fmoc)-Rest derivatisiert ist. Reaktive Seitengruppen der eingesetzten Aminosäuren enthalten Schutzgruppen, die nach Beendigung der Peptidsynthese ohne weiteres abspaltbar sind. Bevorzugte Beispiele hierfür sind Schutzgruppen, wie etwa Triphenylmethyl (Trt), t-Butylether (tBu), t-Butylester (0 tBu), tert.-Butoxycarbonyl (Boc) oder 2,2,5,7,8-Pentamethylchroman-6-sulfonyl (Pmc). Die Aminoseitenketten von Lysinresten oder anderen Aminosäurederivaten mit primären Aminoseitengruppen, die sich an Positionen des Peptids befinden, die später mit dem Hapten derivatisiert werden sollen, sind mit einer ersten Aminoschutzgruppe versehen, die so ausgewählt wird, daß sie quantitativ unter bestimmten Reaktionsbedingungen, z.B. in Anwesenheit von Säure, abspaltbar sind. Ein Beispiel für eine geeignete säurelabile Schutzgruppe ist Boc. Die Seitengruppen von Lysinresten oder anderen Aminosäureresten mit primären Aminoseitengruppen, an denen keine Kopplung eines Haptens gewünscht wird, sind mit einer zweiten Aminoschutzgruppe versehen, die so ausgewählt wird, daß sie unter den Bedingungen, bei denen die erste Schutzgruppe abspaltbar ist, selbst nicht abgespalten wird. Vorzugsweise ist die zweite Schutzgruppe auch unter denjenigen Bedingungen stabil, bei denen die Abspaltung des Peptids von der Festphase und die Abspaltung aller anderen Schutzgruppen erfolgt. Beispiele für solche zweiten Schutzgruppen sind säurestabile Schutzgruppen, wie etwa Phenylacetyl. Neben den 20 natürlichen Aminosäuren kann das Peptid auch artefizielle Aminosäuren, wie etwa β-Alanin, γ-Aminobuttersäure, ∈-Aminocapronsäure, Norleucin oder Ornithin enthalten. Diese artefiziellen Aminosäuren werden analog wie die natürlichen Aminosäuren für die Synthese in geschützter Form eingesetzt.

Nach Beendigung der Synthese erfolgt gegebenenfalls nach Freisetzung des Peptids von der Festphase eine Abspaltung von Schutzgruppen einschließlich der ersten Aminoschutzgruppen, die sich an den Positionen befinden, an denen die Kopplung des Haptens stattfinden soll. Dann wird das auf diese Weise erhaltene Produkt gereinigt, vorzugsweise durch HPLC. Anschließend erfolgt die Einführung der Hapten-Markierung durch Umsetzung des Peptids mit dem jeweils gewünschten Hapten-Aktivesterderivat, das mit freien primären Aminogruppen, d.h. mit der Amino-Endgruppe oder/und Aminoseitengruppen des Peptids, reagiert. Pro freie primäre Aminogruppe werden vorzugsweise 1,5 bis 2,5 Äquivalente Aktivester eingesetzt. Anschließend wird das Reaktionsprodukt aufgereinigt, vorzugsweise durch HPLC.

Enthält das Peptid noch Aminogruppen, die mit einer zweiten Schutzgruppe, wie etwa Phenylacetyl, derivatisiert sind, so werden diese Schutzgruppen im letzten Schritt entfernt. Die Entfernung von Phenylacetylschutzgruppen kann beispielsweise enzymatisch mit immobilisierter oder löslicher Penicillin G-Amidase in wäßriger Lösung mit organischem Solvensanteil bei Raumtemperatur erfolgen.

Enthalten die durch das erfindungsgemäße Verfahren hergestellten Peptide eine intramolekulare Disulfidbrücke, so kann die Peptidsequenz nach Beendigung der Synthese, aber vor Abspaltung der N-terminalen Fmoc-Schutzgruppe der letzten Aminosäure, z.B. mit Jod in Hexafluorisopropanol/Dichlormethan (Kamber und Hiskey in Gross E. und Meienhofer J., The Peptides, Academic Press, New York, 1981, Seiten 145 bis 147) an der Festphase oxidiert, und anschließend die N-terminale Fmoc-Schutzgruppe abgespalten werden.

Es wird ein Peptid synthetisiert, das einen immunologisch reaktiven Epitopbereich, d.h. eine Antikörper-bindende Peptidsequenz, und einen immunologisch nicht reaktiven Spacerbereich umfaßt. Dabei wird mindestens eine Hapten-Markierung an den Spacerbereich gekoppelt. Peptide, bei denen die Markierung im Spacerbereich angeordnet ist, zeigen oft eine bessere Sensitivität in immunologischen Tests.

Der Spacerbereich, der vorzugsweise eine Länge von 1 bis 10 Aminosäuren aufweist, wirkt stabilisierend und löslichkeitsvermittelnd, da er vorzugweise Ladungen enthält oder/und Wasserstoffbrücken ausbilden kann. Außerdem kann er die Bindung von mehreren, z.B. hochmolekularen Rezeptoren an das Hapten-markierte Peptid sterisch erleichtern. Die Aminosäuren des Spacerbereichs werden vorzugsweise ausgewählt aus der Gruppe bestehend aus Glycin, β-Alanin, γ-Aminobuttersäure, ∈-Aminocapronsäure, Lysin und Verbindungen der Strukturformel NH₂-[(CH₂)ₙO)ₓ-CH₂-CH₂-COOH, worin n 2 oder 3 ist und x 1 bis 10 ist. Weiterhin enthält der Spacerbereich vorzugsweise mindestens teilweise artefizielle Aminosäurederivate. Der Spacerbereich ist vorzugsweise am Aminoterminus oder/und Carboxyterminus des Peptids angeordnet.

Durch das erfindungsgemäße Verfahren werden vorzugsweise Peptide synthetisiert, die einen Epitopbereich aus pathogenen Organismen, z.B. Bakterien, Viren und Protozoen, oder aus Autoimmun-Antigenen enthalten. Vorzugsweise stammt der immunologisch reaktive Epitopbereich aus viralen Antigenen, z.B. den Aminosäuresequenzen von HIV I, HIV II, HIV Subtyp O oder Hepatitis C-Virus (HCV).

Vorzugsweise werden HIV I-, HIVII- bzw. HIV Subtyp O-Epitope aus den Regionen gp32, gp41 und gp120 ausgewählt. HCV-Epitope werden vorzugsweise aus der Core/Env-Region oder den Nicht-Strukturprotein-Regionen NS3, NS4 oder NS5 ausgewählt.

Besonders bevorzugt wird der Epitopbereich von HIV I, HIV II-oder HIV Subtyp O-Aminosäuresequenzen ausgewählt aus der Gruppe der Aminosäuresequenzen:

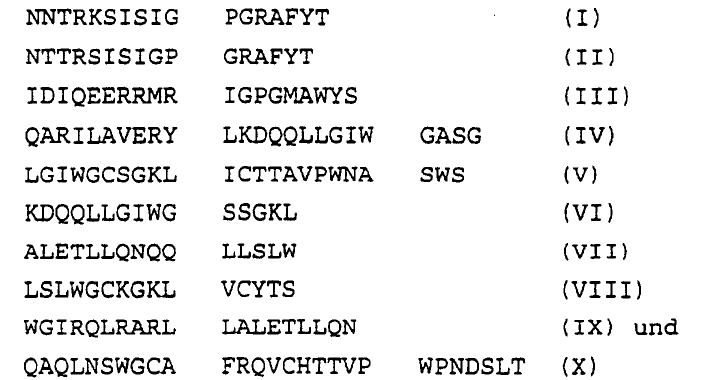

oder Teilsequenzen davon, die eine Länge von mindestens 6 und vorzugsweise mindestens 8 Aminosäuren aufweisen.

Die Aminosäurensequenzen I bis III stammen aus der gp120-Region von HIVI, die Aminosäuresequenzen IV bis IX stammen aus der gp41-Region von HIVI und die Aminosäuresequenz X stammt aus der gp32-Region von HIV II. Die Aminosäuresequenzen I bis X sind weiterhin in den Sequenzprotokollen SEQ ID NO. 1 bis SEQ ID NO. 10 dargestellt. Die Sequenzen V, VIII und X enthalten jeweils 2 Cysteine, die vorzugsweise in Form einer Disulfidbrücke vorliegen. Vorzugsweise enthalten diese Sequenzen einen N-oder/und C-terminalen Spacer, wie oben definiert, der eine Hapten-Markierung, vorzugsweise eine Digoxigenin oder Digoxin, und besonders bevorzugt eine Digoxigenin-3-carboxymethylether-Markierung trägt. Gegebenenfalls können auch innerhalb des Epitopbereichs liegende Lysinreste in markierter Form vorliegen.

Der Epitopbereich von HCV-Aminosäuresequenzen wird vorzugsweise ausgewählt aus der Gruppe der Aminosäuresequenzen:

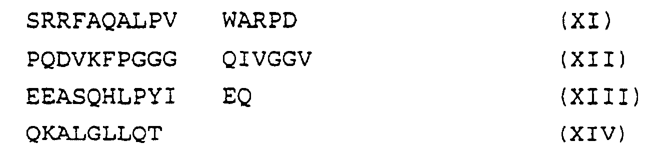

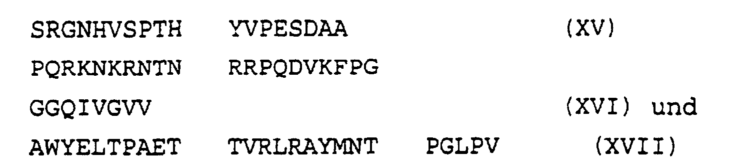

oder Teilsequenzen davon, die eine Länge von mindestens 6 und vorzugsweise mindestens 8 Aminosäuren aufweisen. Die Sequenz XI stammt aus der NS5-Region, die Sequenzen XII und XVI aus der Core Region, die Sequenzen XIII, XIV und XV aus der NS4 Region und die Sequenz XVII aus der NS3 Region von HCV. Die Aminosäuresequenzen XI bis XVII sind in den Sequenzprotokollen SEQ ID NO. 11 bis SEQ ID NO. 17 dargestellt. Vorzugsweise enthalten Peptide mit den oben genannten Epitopen zusätzlich einen Spacerbereich, der eine Hapten-Markierung trägt.

Ein weiterer Gegenstand der vorliegenden Erfindung ist ein Hapten-markiertes Peptid, das eine Länge von maximal 50 und vorzugsweise maximal 30 Aminosäuren aufweist und am Aminoterminus oder/und an Aminoseitengrupen mit mindestens einem Hapten-Aktivesterderivat gekoppelt ist. Vorzugsweise ist das Hapten Digoxigenin oder Digoxin, und der Aktivester ein N-Hydroxysuccinimidester.

Das erfindungsgemäße Peptid umfaßt vorzugsweise einen immunologisch reaktiven Epitopbereich, der mit Antikörpern, z.B. aus Humanseren reagieren kann, und einen immunologisch nicht reaktiven Spacerbereich, wobei der Spacerbereich mindestens eine Haptenmarkierung trägt. Vorzugsweise ist der Spacerbereich am Aminoterminus des Peptids angeordnet, und hat eine Länge von 1 bis 10 Aminosäuren. Der Epitopbereich stammt vorzugsweise aus den Aminosäuresequenzen von HIV I, HIV II oder HCV einschließlich Varianten, z.B. Subtypen davon, z.B. HIV Subtyp O, und ist eine der Aminosäuresequenzen I bis XVII oder eine Teilsequenz davon.

Die vorliegende Erfindung betrifft auch die Verwendung von Hapten-markierten Peptiden als Antigene bei einem immunologischen Verfahren zur Bestimmung von spezifischen Antikörpern in einer Probeflüssigkeit. Vorzugsweise werden solche Antikörper bestimmt, die auf eine Infektion durch Mikroorganismen, wie etwa Bakterien, Viren oder Protozoen, hinweisen. Besonders bevorzugt werden gegen Viren gerichtete Antikörper, z.B. gegen HIV oder Hepatitis-Viren gerichtete Antikörper bestimmt. Die Probeflüssigkeit ist vorzugsweise Serum, besonders bevorzugt humanes Serum. Weiterhin ist bevorzugt, daß die erfindungsgemäßen Hapten-markierten Peptide bei einem immunologischen Verfahren im Brückentestformat eingesetzt werden.

Außerdem betrifft die vorliegende Erfindung ein Verfahren zur immunologischen Bestimmung eines spezifischen Antikörpers in einer Probeflüssigkeit, welches dadurch gekennzeichnet ist, daß man die Probeflüssigkeit mit (a) einem ersten markierten Antigen, das gegen den zu bestimmenden Antikörper gerichtet ist und ein Hapten-markiertes Peptid, wie oben definiert, umfaßt, und (b) einem Rezeptor für das Hapten, der eine signalerzeugende Gruppe trägt, inkubiert und den Antikörper über eine Bindung mit dem Peptid nachweist. Vorzugsweise verwendet man als erstes Antigen ein mit Digoxin oder Digoxigenin markiertes Peptid, und als Rezeptor einen gegen Digoxigenin oder/und Digoxin gerichteten Antikörper. Der Begriff "Antikörper" soll in diesem Zusammenhang auch Antikörperfragmente, z.B. Fab-, Fab' -, F(ab)₂-, F(ab')₂-Fragmente oder andere z.B. gentechnologisch modifizierte Antikörperfragmente, umfassen. Der Rezeptor ist mit einer signalerzeugenden Gruppe, vorzugsweise einem Enzym, wie etwa Peroxidase, alkalische Phosphatase, β-Galactosidase, Urease oder Q-β-Replikase, gekoppelt. Die signalerzeugende Gruppe kann jedoch auch eine chromogene, radioaktive oder NMR-aktive Gruppe oder ein Metallpartikel (z.B. Gold) sein. Vorzugsweise ist die signalerzeugende Gruppe ein Enzym.

Das erfindungsgemäße immunologische Bestimmungsverfahren kann an sich nach jedem bekannten Testformat erfolgen, z.B. in einem homogenen Immunoassay mit einer einzigen Reaktionsphase oder in einem heterogenen Immunoassay mit mehr als einer Reaktionsphase. vorzugsweise wird ein heterogenes Testformat verwendet, bei dem das Vorhandensein des Antikörpers in Anwesenheit einer Festphase nachgewiesen wird. Eine Ausführungsform dieses Testformats ist das sogenannte Doppelantigen-Brückentestkonzept. Hierbei wird die Probeflüssigkeit in Gegenwart einer Festphase mit dem ersten Antigen und einem zweiten Antigen inkubiert, das gegen den zu bestimmenden Antikörper gerichtet ist und (a) an die Festphase gebunden ist, oder (b) in einer an die Festphase bindefähigen Form vorliegt. Der zu bestimmende Antikörper in der Probeflüssigkeit wird durch Bestimmung der Markierung in der Festphase oder/und in der flüssigen Phase nachgewiesen. Vorzugsweise ist das zweite Antigen mit Biotin markiert und ist an eine Festphase bindefähig, die mit Streptavidin oder Avidin beschichtet ist. Vorzugsweise verwendet man als zweites Antigen ein mit Biotin markiertes Peptid.

Die Testdurchführung beinhaltet vorzugsweise ein Mischen der Probeflüssigkeit mit dem ersten Antigen und dem festphasenseitigen zweiten Antigen, um einen markierten, immobilisierten Komplex aus erstem Antigen, Antikörper und festphasengebundenem zweiten Antigen zu erhalten. Gegenüber anderen Testformaten zum Nachweis von Antikörpern führt das Brückentestformat sowohl zu einer Verbesserung der Sensitivität, d.h. es werden alle Immunglobulinklassen, wie etwa IgG, IgM, IgA und IgE, erkannt, als auch der Spezifität, d.h. es wird die unspezifische Reaktivität verringert. Die Spezifität und Sensitivität des Doppelantigen-Brückentests kann weiterhin verbessert werden, wenn man eine Zweischritt-Testführung verwendet, bei der in einem ersten Schritt die Probeflüssigkeit mit dem ersten und dem zweiten Antigen vermischt, und anschließend der Rezeptor für die Haptenmarkierung des ersten Antigens, der die signalerzeugende Gruppe trägt, zugegeben wird.

Ein weiterer Vorteil des Doppelantigen-Brückentestformats, bei dem ein festphasenseitiges und ein Hapten-markiertes Peptid als Antigene eingesetzt werden, besteht in der Möglichkeit der Verringerung des Risikos einer falsch negativen Bewertung von Proben, die einen hohen Titer des zu bestimmenden Antikörpers aufweisen, infolge des Hook-Effekts und zwar durch eine Erhöhung der Anzahl von Markierungsgruppen pro Peptid vorzugsweise auf 2 bis 10 Markierungsgruppen. Die Erhöhung der Anzahl von Haptenmarkierungsgruppen pro Peptid führt infolge der Amplifikation des Signals über den Rezeptor zur Verbesserung der Hook-Sensitivität gegenüber Testführungen mit direkt nachweisbaren Markierungsgruppen.

Noch ein weiterer Gegenstand der vorliegenden Erfindung ist ein Reagenz zur immunologischen Bestimmung eines spezifischen Antikörpers, das mindestens ein erfindungsgemäßes Hapten-markiertes, mit dem zu bestimmenden Antikörper reagierendes Peptid enthält. Wird das Reagenz in einem Doppelantigen-Brückentest verwendet, so enthält es vorzugsweise (a) das Hapten-markierte Peptid, (b) einen Rezeptor für das Hapten, der eine signalerzeugende Gruppe trägt, und (c) ein weiteres, mit dem zu bestimmenden Antikörper reagierendes Antigen, das an eine Festphase gebunden ist oder in einer an eine Festphase bindefähigen Form vorliegt. Das Hapten ist vorzugsweise ein Cardenolid oder Cardenolid-Glycosid, insbesondere Digoxin oder Digoxigenin, der Rezeptor für das Hapten ist vorzugsweise ein gegen das Hapten gerichteter Antikörper, die signalerzeugende Gruppe ist vorzugsweise ein Enzym, das weitere Antigen ist vorzugsweise biotinyliert und ist an eine mit Streptavidin oder Avidin beschichtete Festphase bindefähig.

Weiterhin wird die vorliegende Erfindung durch die nachfolgenden Beispiele und Sequenzprotokolle beschrieben.

Es zeigen
- SEQ ID NO. 1:: die Aminosäuresequenz eines Epitops aus dem gp120-Bereich von HIV I;
- SEQ ID NO. 2:: die Aminosäuresequenz eines weiteren Epitops aus dem gp120-Bereich von HIV I;
- SEQ ID NO. 3:: die Aminosäuresequenz eines weiteren Epitops aus dem gp120-Bereich von HIV I, Subtyp O;
- SEQ ID NO. 4:: die Aminosäuresequenz des Epitops aus dem gp41-Bereich von HIV I;
- SEQ ID NO. 5:: die Aminosäuresequenz eines weiteren Epitops aus dem gp41-Bereich von HIV I;
- SEQ ID NO. 6:: die Aminosäuresequenz noch eines weiteren Epitops aus dem gp41-Bereich von HIV I;
- SEQ ID NO. 7:: die Aminosäuresequenz eines Epitops aus dem gp41-Bereich von HIV I, Subtyp O;
- SEQ ID NO. 8:: die Aminosäuresequenz eines weiteren Epitops aus dem gp41-Bereich von HIV I, Subtyp O;
- SEQ ID NO. 9:: die Aminosäuresequenz noch eines weiteren Epitops aus dem gp41-Bereich von HIV I, Subtyp O;
- SEQ ID NO.10:: die Aminosäuresequenz eines Epitops aus dem gp32-Bereich von HIV II;
- SEQ ID NO.11:: die Aminosäuresequenz eines Epitops aus dem NS5-Bereich von HCV;
- SEQ ID NO.12:: die Aminosäuresequenz eines Epitops aus dem Core-Bereich von HCV;
- SEQ ID NO.13:: die Aminosäuresequenz eines Epitops aus dem NS4-Bereich von HCV;
- SEQ ID NO.14:: die Aminosäuresequenz eines weiteren Epitops aus dem NS4-Bereich von HCV;
- SEQ ID NO.15:: die Aminosäuresequenz noch eines weiteren Epitops aus dem NS4-Bereich von HCV;
- SEQ ID NO.16:: die Aminosäuresequenz eines weiteren Epitops aus dem Core-Bereich von HCV; und
- SEQ ID NO.17:: die Aminosäuresequenz eines Epitops aus dem NS3-Bereich von HCV.

### Beispiel 1

### Herstellung von Hapten-markierten Peptiden

Die Hapten-markierten Peptide wurden mittels Fluorenylmethyloxycarbonyl-(Fmoc)-Festphasenpeptidsynthese an einem Batch-Peptidsynthesizer, z.B. von Applied Biosystems A431 oder A433, hergestellt. Dazu wurden jeweils 4.0 Äquivalente der in Tabelle 1 dargestellten Aminosäurederivate verwendet:

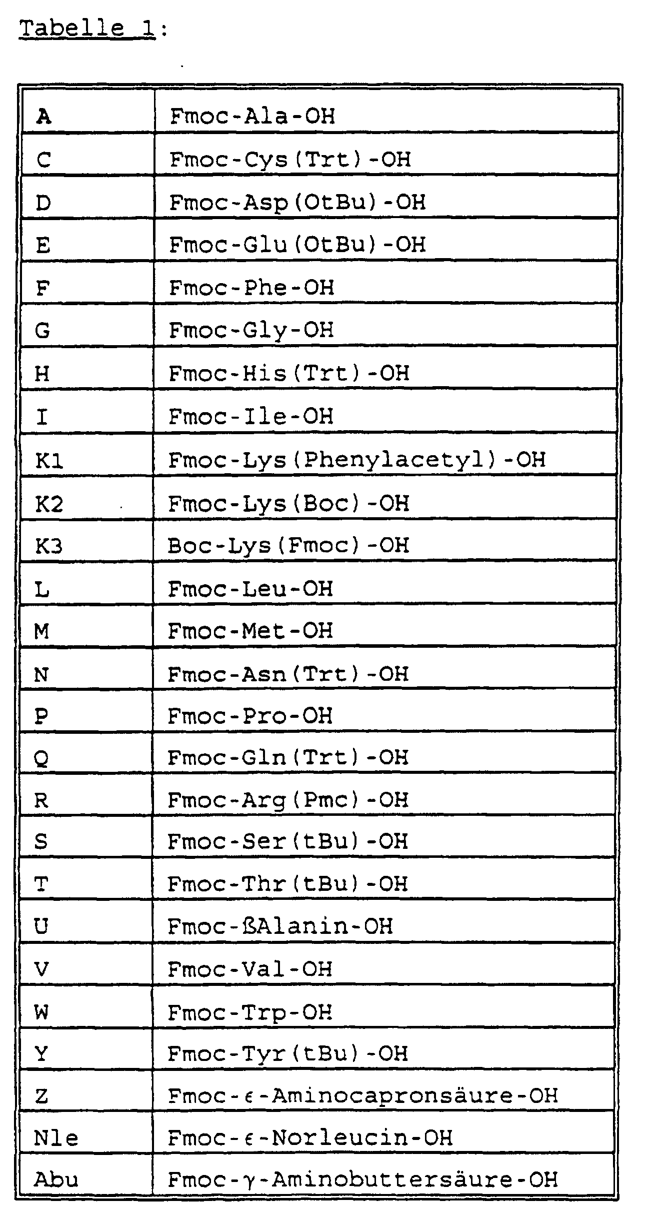

Das Lysin-Derivat K1 wurde für Positionen verwendet, an denen keine Haptenmarkierung eingeführt werden sollte. Das Lysin-Derivat K2 wurde für Positionen verwendet, an denen eine Haptenmarkierung eingeführt werden sollte. Das Lysin-Derivat K3 wurde zur Kopplung der ∈-Aminogruppe an das Peptid im Spacerbereich verwendet.

Die Aminosäuren oder Aminosäurederivate wurden in N-Methylpyrrolidon gelöst. Das Peptid wurde an 400-500 mg 4-(2',4'-Dimethoxyphenyl-Fmoc-Aminomethyl)-Phenoxy-Harz (Tetrahedron Letters 28 (1987), 2107) mit einer Beladung von 0,4-0,7 mmol/g aufgebaut (JACS 95 (1973), 1328). Die Kupplungsreaktionen wurden bezüglich des Fmoc-Aminosäurederivats mit 4 Äquivalenten Dicyclohexylcarbodiimid und 4 Äquivalenten N-Hydroxybenzotriazol in Dimethylformamid als Reaktionsmedium während 20 min durchgeführt. Nach jedem Syntheseschritt wurde die Fmoc-Gruppe mit 20%igem Piperidin in Dimethylformamid in 20 min abgespalten.

Bei Anwesenheit von Cysteinresten in der Peptidsequenz erfolgte unmittelbar nach Beendigung der Synthese eine Oxidation an der Festphase mit Jod in Hexafluorisopropanol/Dichlormethan.

Die Freisetzung des Peptids vom Syntheseharz und die Abspaltung der säurelabilen Schutzgruppen - mit Ausnahme der Phenylacetylschutzgruppe - erfolgte mit 20 ml Trifluoressigsäure, 0,5 ml Ethandithiol, 1 ml Thioanisol, 1,5 g Phenol und 1 ml Wasser in 40 min bei Raumtemperatur. Die Reaktionslösung wurde anschließend mit 300 ml gekühltem Diisopropylether versetzt und zur vollständigen Fällung des Peptids 40 min bei 0°C gehalten. Der Niederschlag wurde abfiltriert, mit Diisopropylether nachgewaschen, mit wenig 50 %-iger Essigsäure gelöst und lyophilisiert. Das erhaltene Rohmaterial wurde mittels präparativer HPLC an Delta-PAK RP C18-Material (Säule 50 x 300 mm, 100 Å, 15 µ) über einen entsprechenden Gradienten (Eluent A: Wasser, 0,1% Trifluoressigsäure, Eluent B: Acetonitril, 0,1% Trifluoressigsäure) in ca. 120 min. aufgereinigt. Die Identität des eluierten Materials wurde mittels Ionenspray-Massenspektrometrie geprüft.

Die Einführung der Hapten-, z.B. einer Digoxigenin- bzw. Digoxin-Markierung erfolgte über entsprechende Aktivester-Derivate and die freien Aminogruppen des Peptids in Lösung. Das zu derivatisierende Peptid wurde in einer Mischung aus DMSO und 0,1 M Kaliumphosphat-Puffer pH 8,5 gelöst. Anschließend wurden 2 Äquivalente Aktivester pro freie primäre Aminofunktion in wenig DMSO gelöst zugetropft und bei Raumtemperatur gerührt. Der Umsatz wurde über analytische HPLC verfolgt. Das Produkt wird mittels präparativer HPLC aufgereinigt.

Enthielt das Peptid noch mit Phenyacetyl geschützte Lysine, so wurde diese Schutzgruppe im letzten Schritt enzymatisch mit Penicillin-G-Amidase in wäßrigem Milieu mit organischem Solvens-Anteil bei Raumtemperatur abgespalten. Das Enzym wurde abgetrennt, z.B. durch Filtration, und das Peptid über präparative HPLC aufgereinigt. Die Identität des eluierten Materials wurde mittels Ionenspray-Massenspektrometrie geprüft.

Aus den Bereichen gp120, gp41 und gp32 von HIV I bzw. HIV II wurden unter Verwendung von Digoxigenin-3-carboxymethylether-N-hydroxysuccinimidester (Boehringer Mannheim GmbH, Mannheim, BRD) die in Tabelle 2 dargestellten Peptidverbindungen hergestellt.

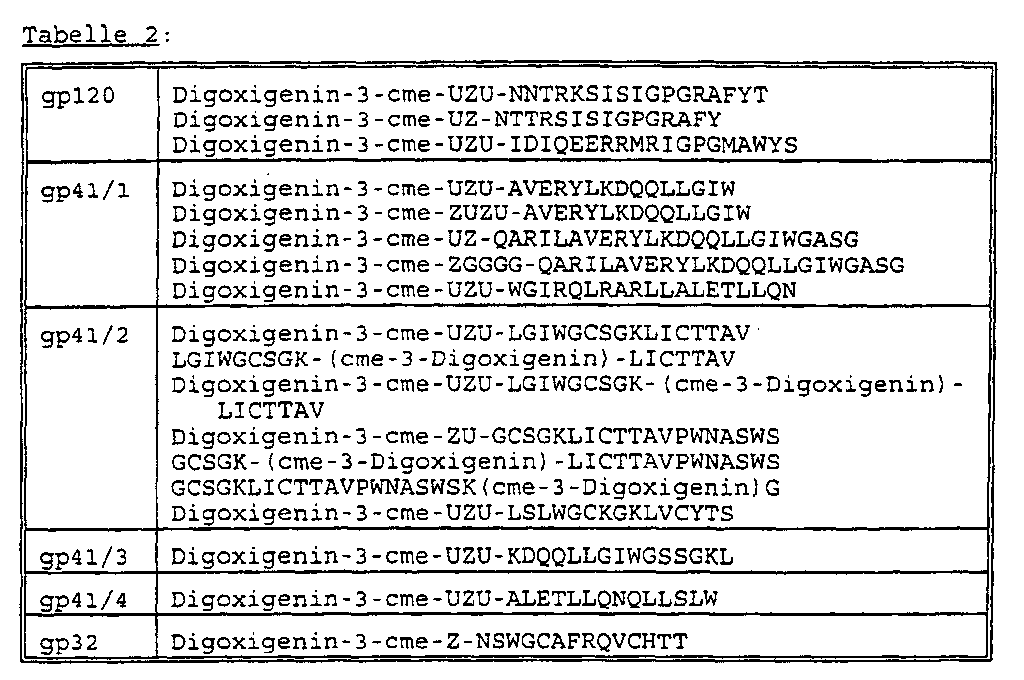

Aus dem NS5-Bereich, dem NS4-Bereich und dem Core-Bereich von HCV wurden die in der folgenden Tabelle 3 dargestellten Peptide synthetisiert.

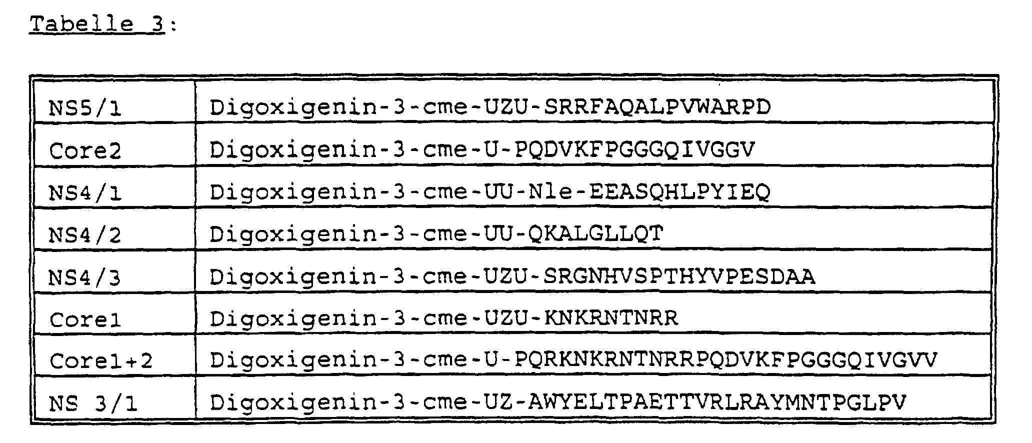

Die Herstellung Biotin-markierter Peptide erfolgte entweder N-terminal durch eine Derivatisierung am Harz (Biotin-Aktivester) oder in die Sequenz über einen mit Biotin-e-derivatisierten Lysinrest (Fmoc-Lys (Biotin)-OH).

### Beispiel 2

### Verbesserung von Spezifität und Sensitivität durch eine bevorzugte Testführung

Die Spezifität und Sensitivität eines Doppelantigen-Brückentests unter Verwendung der erfindungsgemäßen Peptide kann auch durch eine Testführung verbessert werden, bei der in einem ersten Schritt die Probe, das Hapten-markierte Antigen und das festphasenseitige Antigen vermischt und anschließend, vorzugsweise nach 1 bis 4 h, besonders bevorzugt nach 1,5 bis 2,5 h, der Anti-Hapten-Antikörper zugesetzt wird.

Als Antigene wurden die HIV-Epitope gp41/1 und gp41/2 (Tabelle 2) verwendet.

Die Testbedingungen für den bevorzugten Zweischritt-Test waren wie folgt:
- neutraler Kaliumphosphatpuffer 50mmol/l, pH 7,2, 0,2% Rinderserumalbumin (RSA), 0,2 % Natriumlaurylsulfat (SLS)-Detergenz
- Inkubationszeiten
   - Inkubation von Hapten-markiertem und festphasenseitigem Antigen mit Serum: 120 min
   - Inkubation mit Konjugat aus Anti-Digoxigenin-Antikörper und Peroxidase (<Dig>-POD) 60 min
   - Inkubation mit 2,2'-Azino-di-[3-ethylbenzylthiazolin-Sulfonat(6)] (ABTS): 60 min
- Inkubationstemperatur: 25 °C
- bound/free-Trennung zwischen allen Inkubationsschritten

Die Testbedingungen für den alternativen Zweischritt-Test waren wie folgt:
- neutraler Kaliumphosphatpuffer 50 mmol/l, pH 7,2, 0,2 % RSA, 0,2 % SLS-Detergenz
- Inkubationszeiten
   - Inkubation von festphasenseitigem Antigen mit Serum: 90 min
   - Inkubation mit Hapten-markiertem Antigen und <Dig>-POD: 90 min
   - Inkubation mit ABTS: 60 min
- Inkubationstemperatur: 25 °C
- bound/free-Trennung zwischen allen Inkubationsschritten

Die Testbedingungen für den Einschritt-Test waren wie folgt:
- neutraler Kaliumphosphatpuffer 50 mmol/l, pH 7,2, 0,2 % RSA, 0,2 % SLS-Detergenz
- Inkubationszeiten
   - Inkubation beide Antigen mit Serum und <Dig>-POD 120 min
   - Inkubation mit ABTS: 60 min
- Inkubationstemperatur: 25 °C
- bound/free-Trennung zwischen allen Inkubationsschritten

Die Ergebnisse des Tests sind in der Tabelle 4 dargestellt. Es ist zu erkennen, daß bei der bevorzugten Testführung eine viel höhere Signaldifferenzierung, d.h. ein Verhältnis der Meßsignale von positiven Proben zu negativen Proben, erreicht wird.

**Tabelle 4**

| Probennummer | Einschritt Testführung | bevorzugte ZweischrittTestführung | alternative Zweischritt-Testführung |
|---|---|---|---|
| | | | |
| | | 1. Mischen von Probe u. beiden spezifischen Antigenen | 1. Mischen von Probe u. wandseitigem spezifischem Antigen |
| | | | |
| | | 2. Zusatz von von Anti-Hapten-Anti-körper für die Nachweisreaktion | 2. Zusatz von detektionsseitigem spezifischen Antigen und Anti-Hapten-Antikörper für die Nachweisreaktion |

| A) negative Proben | Signal in mE | Signal in mE | Signal in mE |
|---|---|---|---|
| 1 | 19 | 7 | 7 |
| 2 | 21 | 12 | 9 |
| 3 | 17 | 6 | 5 |
| 4 | 19 | 5 | 7 |
| 5 | 18 | 4 | 8 |
| 6 | 28 | 7 | 16 |
| 7 | 20 | 10 | 9 |
| 8 | 21 | 7 | 11 |
| 9 | 18 | 10 | 7 |
| 10 | 19 | 11 | 8 |
| 11 | 17 | 8 | 9 |
| 12 | 19 | 12 | 7 |
| 13 | 22 | 7 | 7 |
| 14 | 20 | 5 | 17 |
| 15 | 24 | 40 | 8 |
| 16 | 19 | 10 | 7 |
| 17 | 20 | 4 | 8 |
| 18 | 23 | 6 | 8 |
| 19 | 20 | 8 | 7 |
| 20 | 16 | 11 | 7 |

| B) positive Proben | Signal in mE | Signal in mE | Signal in mE |
|---|---|---|---|
| 1 | 405 | 2401 | 3681 |
| 2 | 1080 | 4836 | 4931 |
| 3 | 158 | 1100 | 300 |
| 4 | 760 | 6210 | 2155 |
| 5 | 1094 | 3578 | 1835 |
| 6 | 452 | 2296 | 2954 |
| 7 | 163 | 1068 | 136 |
| 8 | 76 | 195 | 14 |
| 9 | 2405 | 7803 | 2671 |
| 10 | 293 | 3093 | 575 |
| 11 | 303 | 2430 | 42 |
| 12 | 37 | 132 | 11 |
| 13 | 19 | 9 | 9 |
| 14 | 63 | 218 | 11 |
| 15 | 74 | 297 | 15 |
| 16 | 60 | 253 | 16 |
| 17 | 86 | 509 | 17 |
| 18 | 106 | 1182 | 22 |
| 19 | 962 | 8782 | 338 |
| 20 | 815 | 7335 | 167 |

### Beispiel 3

In einem Doppelantigen-Brückentest wurde ein erfindungsgemäßes Peptidantigen mit einem rekombinanten Polypeptidantigen verglichen. In einem erfindungsgemäßen Beispiel wurde das digoxigenylierte Peptidantigen gp41/2 (Tabelle 2) in Kombination mit einem biotinylierten Peptidantigen der gleichen Sequenz getestet. In einem Vergleichsbeispiel wurde ein digoxigenyliertes Polypeptidantigen rec. gp41 (Chang et al., Science 228 (1985), 93-96) in Kombination mit einem biotinylierten Polypeptidantigen der gleichen Sequenz getestet.

Die Ergebnisse des Tests sind in Tabelle 5 dargestellt. "NK" bedeutet negative Kontrolle, "PK" bedeutet positive Kontrolle. Der "cut-off" Index ist die Grenze zwischen positiver und negativer Bewertung eines Experiments. Er ist als 2x NK definiert. Aus Tabelle 5 ist ersichtlich, daß mit dem rekombinanten Polypeptidantigen praktisch keine Differenzierung zwischen negativen und positiven Proben möglich ist, während das Peptidantigen eine sehr gute Differenzierung erlaubt.

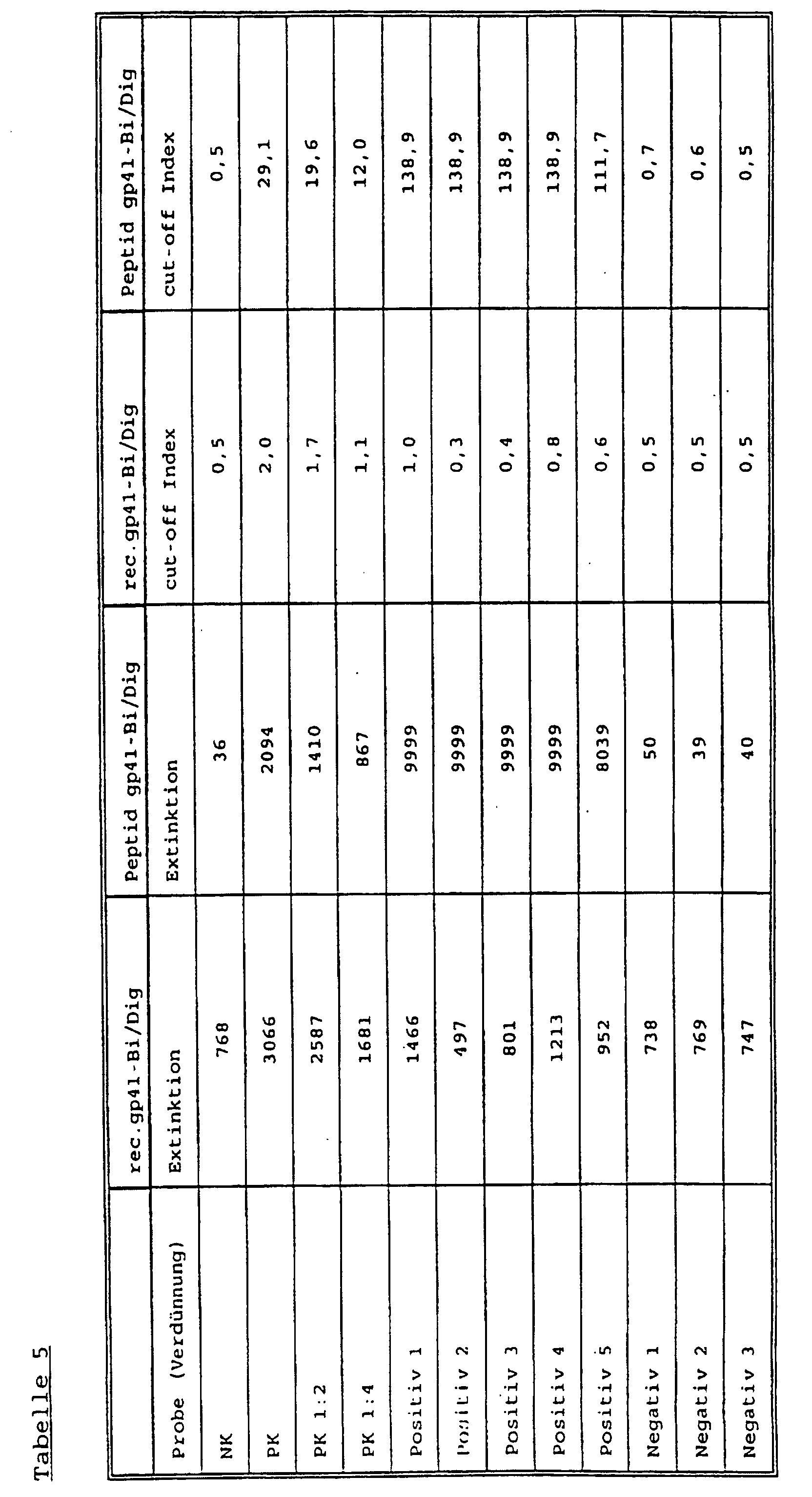

## Patentansprüche

1. Verfahren zur Herstellung von Hapten-markierten Peptiden,
**dadurch gekennzeichnet,**
**daß** man
(a) ein Peptid mit der gewünschten Aminosäuresequenz an einer Festphase aus Aminosäurederivaten synthetisiert, deren reaktive Seitengruppen durch Schutzgruppen blockiert sind, wobei die Schutzgruppen an primären Aminoseitengruppen derart ausgewählt werden, daß sie gegebenenfalls selektiv abspaltbar sind,
(b) eine Abspaltung von Schutzgruppen durchführt, wobei mindestens eine freie primäre Aminogruppe entsteht,
(c) ein Hapten-Aktivesterderivat an die mindestens eine freie primäre Aminogruppe des Peptids koppelt, und
(d) gegebenenfalls eine Abspaltung von noch verbleibenden Schutzgruppen durchführt,
wobei das Hapten ausgewählt wird aus der Gruppe bestehend aus Sterinen, Gallensäuren, Sexualhormonen, Corticoiden, Cardenoliden, Cardenolid-Glycosiden, Bufadienoliden, Steroid-Sapogeninen und Steroidalkaloiden.

2. Verfahren nach Anspruch 1,
**dadurch gekennzeichnet,**
**daß** das Hapten ausgewählt wird aus der Gruppe bestehend aus Cardenoliden und Cardenolid-Glycosiden.

3. Verfahren nach Anspruch 2,
**dadurch gekennzeichnet,**
**daß** das Hapten ausgewählt wird aus der Gruppe bestehend aus Digoxigenin, Digitoxigenin, Gitoxigenin, Strophantidin, Digoxin, Digitoxin, Ditoxin und Strophantin.

4. Verfahren nach Anspruch 3,
**dadurch gekennzeichnet,**
**daß** man Digoxigenin oder Digoxin als Hapten verwendet.

5. Verfahren nach einem der Ansprüche 1 bis 4,
**dadurch gekennzeichnet,**
**daß** man an Positionen des Peptids, an denen eine Kopplung des Haptens erfolgen soll, ein Aminosäurederivat mit einer ersten Schutzgruppe für die Aminoseitenkette verwendet, die unter bestimmten Reaktionsbedingungen quantitativ abspaltbar ist, und daß man an Positionen des Peptids, an denen keine Kopplung des Haptens erfolgen soll, ein Aminosäurederivat mit einer zweiten Schutzgruppe für die Aminoseitenkette verwendet, die unter den Reaktionsbedingungen, bei denen die erste Schutzgruppe abgespalten wird, selbst nicht abspaltbar ist.

6. Verfahren nach Anspruch 5,
**dadurch gekennzeichnet,**
**daß** man eine erste säurelabile Schutzgruppe und eine zweite säurestabile Schutzgruppe verwendet.

7. Verfahren nach einem der Anspruche 1 bis 6,
**dadurch gekennzeichnet,**
**daß** man als Aktivesterderivat einen N-Hydroxysuccinimidester verwendet.

8. Verfahren nach einem der Ansprüche 1 bis 7,
**dadurch gekennzeichnet,**
**daß** man ein Peptid synthetisiert, das einen immunologisch reaktiven Epitopbereich und einen Spacerbereich umfaßt,
wobei mindestens eine Hapten-Markierung an den Spacerbereich gekoppelt wird.

9. Verfahren nach Anspruch 8,
**dadurch gekennzeichnet,**
**daß** der Spacerbereich eine Länge von 1 bis 10 Aminosäuren aufweist.

10. Verfahren nach Anspruch 8 oder 9,
**dadurch gekennzeichnet,**
**daß** ein Spacerbereich am Amino- oder/und Carboxyterminus des Peptids angeordnet ist.

11. Verfahren nach einem der Ansprüche 8 bis 10,
**dadurch gekennzeichnet,**
**daß** der Spacerbereich Aminosäuren enthält, die Ladungen aufweisen oder/und Wasserstoffbrücken ausbilden können.

12. Verfahren nach einem der Anprüche 8 bis 11,
**dadurch gekennzeichnet,**
**daß** die Aminosäuren des Spacerbereichs ausgewählt werden aus der Gruppe bestehend aus Glycin, β-Alanin, γ-Aminobuttersäure, ∈-Aminocapronsäure, Lysin und Verbindungen der Strukturformel NH₂-[(CH₂)ₙ-O]ₓ-CH₂-CH₂-COOH, worin n 2 oder 3 ist und x 1 bis 10 ist.

13. Verfahren nach einem der Ansprüche 1 bis 12,
**dadurch gekennzeichnet,**
**daß** man ein Peptid synthetisiert, das einen Epitopbereich aus den Aminosäuresequenzen von HIV I, HIV II oder HCV enthält.

14. Verfahren nach Anspruch 13,
**dadurch gekennzeichnet,**
**daß** der Epitopbereich ausgewählt wird aus der Gruppe der HIV I- oder HIV II-Aminosäuresequenzen 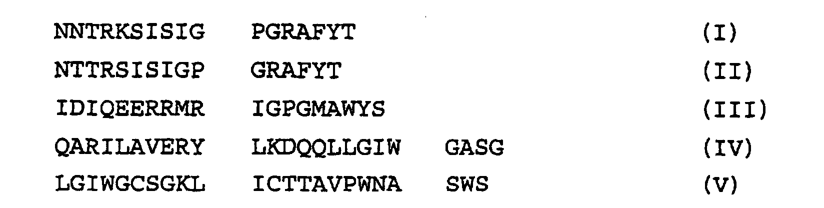 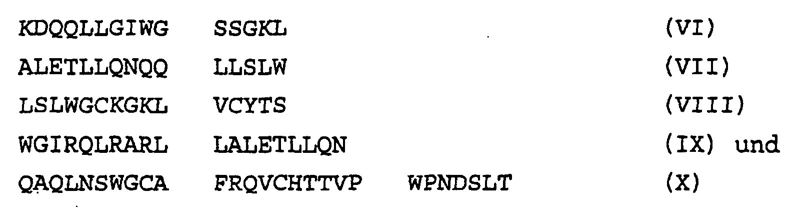 oder Teilsequenzen davon, die eine Länge von mindestens 6 Aminosäuren aufweisen.

15. Verfahren nach Anspruch 13,
**dadurch gekennzeichnet,**
**daß** der Epitopbereich ausgewählt wird aus der Gruppe der HCV-Aminosäuresequenzen 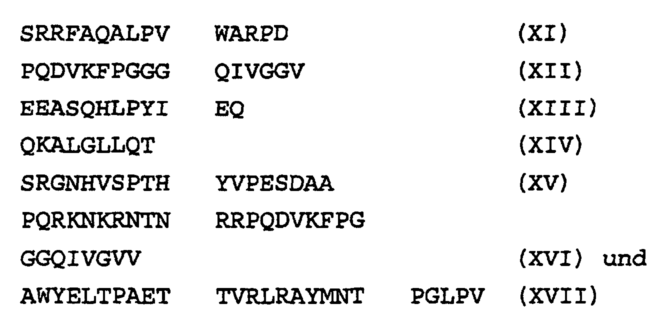 oder Teilsequenzen davon, die eine Länge von mindestens 6 Aminosäuren aufweisen.

16. Hapten-markiertes Peptid, das eine Länge von maximal 50 Aminosäuren aufweist und am Aminoterminus oder/und an Aminoseitengruppen mit mindestens einem Hapten-Aktivester-derivat gekoppelt ist, wobei das Hapten ausgewählt ist aus der Gruppe bestehend aus Sterinen, Gallensäuren, Sexualhormonen, Corticoiden, Cardenoliden, Cardenolid-Glycosiden, Bufadienoliden, Steroid-Sapogeninen und Steroidalkaloiden, und wobei
das Peptid einen immunologisch reaktiven Epitopbereich und einen immunologisch nicht reaktiven Spacerbereich umfaßt, wobei der Spacerbereich mindestens eine Hapten-Markierung trägt.

17. Peptid nach Anspruch 16,
**dadurch gekennzeichnet,**
**daß** das Hapten Digoxigenin oder Digoxin ist.

18. Peptid nach Anspruch 16,
**dadurch gekennzeichnet,**
**daß** ein Spacerbereich am Amino- oder/und Carboxyterminus des Peptids angeordnet ist.

19. Peptid nach einem der Ansprüche 16 bis 18,
**dadurch gekennzeichnet,**
**daß** der Epitopbereich aus den Aminosäuresequenzen von IV I, HIV II oder HCV stammt.

20. Peptid nach Anspruch 19,
**dadurch gekennzeichnet,**
**daß** der Epitopbereich ausgewählt ist aus der Gruppe der HIV I oder HIV II-Aminosäuresequenzen 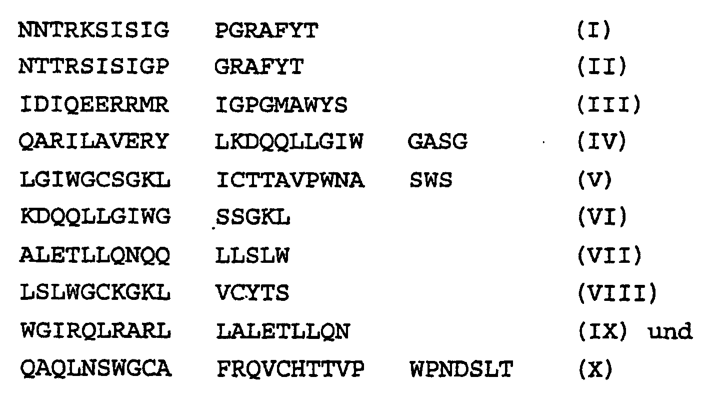 oder Teilsequenzen davon, die eine Länge von mindestens 6 Aminosäuren aufweisen.

21. Peptid nach Anspruch 19,
**dadurch gekennzeichnet,**
**daß** der Epitopbereich ausgewählt ist aus der Gruppe der HCV-Aminosäuresequenzen 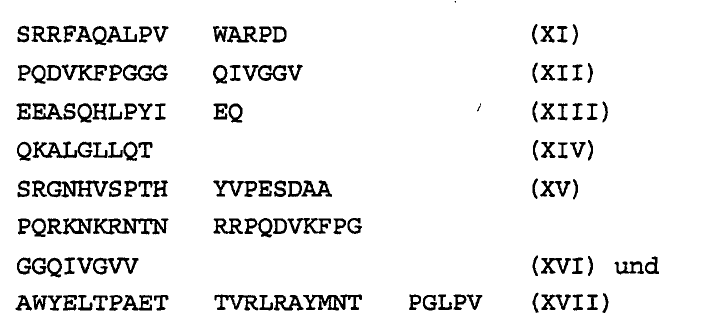 oder Teilsequenzen davon, die eine Länge von mindestens 6 Aminosäuren aufweisen.

22. Verwendung von Hapten-markierten Peptiden, die durch ein Verfahren nach einem der Ansprüche 1 bis 15 hergestellt wurden, oder von Peptiden nach einem der Ansprüche 16 bis 21 als Antigene bei einem immunologischen Verfahren zur Bestimmung von spezifischen Antikörpern in einer Probeflüssigkeit.

23. Verwendung nach Anspruch 22 bei einem immunologischen Verfahren im Brückentestformat.

24. Verfahren zur immunologischen Bestimmung eines spezifischen Antikörpers in einer Probeflüssigkeit
**dadurch gekennzeichnet,**
**daß** man die Probeflüssigkeit mit
(a) einem ersten markierten Antigen, das gegen den zu bestimmenden Antikörper gerichtet ist und ein Hapten-markiertes Peptid, das durch ein Verfahren nach einem der Ansprüche 1 bis 15 hergestellt wurde, oder ein Peptid nach einem der Ansprüche 16 bis 21 umfaßt, und
(b) einem Rezeptor für das Hapten, der eine signalerzeugende Gruppe trägt,
inkubiert und den Antikörper über eine Bindung mit dem Peptid nachweist.

25. Verfahren nach Anspruch 24,
**dadurch gekennzeichnet,**
**daß** man als erstes Antigen ein mit Digoxin oder Digoxigenin markiertes Peptid und als Rezeptor einen gegen Digoxigenin oder/und Digoxin gerichteten Antikörper verwendet.

26. Verfahren nach Anspruch 24 oder 25,
**dadurch gekennzeichnet,**
**daß** man die Probeflüssigkeit in Gegenwart einer Festphase mit dem ersten Antigen und einem zweiten Antigen inkubiert, das gegen den zu bestimmenden Antikörper gerichtet ist und
(a) an die Festphase gebunden ist oder
(b) in einer an die Festphase bindefähigen Form vorliegt, und den zu bestimmenden Antikörper durch Bestimmung der Markierung in der Festphase oder/und in der flüssigen Phase nachweist.

27. Verfahren nach Anspruch 26,
**dadurch gekennzeichnet,**
**daß** man als zweites Antigen ein mit Biotin markiertes Antigen und eine Festphase, die mit Streptavidin oder Avidin beschichtet ist, verwendet.

28. Verfahren nach Anspruch 27,
**dadurch gekennzeichnet,**
**daß** man als zweites Antigen ein mit Biotin markiertes Peptid verwendet.

29. Verfahren nach einem der Ansprüche 26 bis 28,
**dadurch gekennzeichnet,**
**daß** man die Probeflüssigkeit mit dem ersten und dem zweiten Antigen vermischt und anschließend den Rezeptor für das Hapten des ersten Antigens zusetzt.

30. Reagenz zur immunologischen Bestimmung eines spezifischen Antikörpers,
**dadurch gekennzeichnet,**
**daß** es mindestens ein Hapten-markiertes, mit dem zu bestimmenden Antikörper reagierendes Peptid, das durch ein Verfahren nach einem der Ansprüche 1 bis 15 hergestellt wurde, oder ein Hapten-markiertes, mit dem zu bestimmenden Antikörper reagierendes Peptid nach einem der Ansprüche 16 bis 21 enthält.

31. Reagenz nach Anspruch 30,
**dadurch gekennzeichnet,**
**daß** es
(a) das Hapten-markierte, mit dem zu bestimmenden Antikörper reagierende Peptid,
(b) einen Rezeptor für das Hapten, der eine signalerzeugende Gruppe trägt, und
(c) ein weiteres, mit dem zu bestimmenden Antikörper reagierendes Antigen, das an eine Festphase gebunden ist oder in einer an eine Festphase bindefähige Form vorliegt, enthält.

## Claims

1. Process for producing hapten-labelled peptides,
**characterized in that**
(a) a peptide having the desired amino acid sequence is synthesized on a solid phase from amino acid derivatives whose reactive side groups are blocked by protective groups, the protective groups on primary amino side groups being selected such that they can be optionally selectively removed by cleavage,
(b) protective groups are cleaved off to form at least one free primary amino group,
(c) a hapten-active ester derivative is coupled to the at least one free primary amino group of the peptide and
(d) any remaining protective groups are optionally cleaved off,
the hapten being selected from the group comprising sterols, gallic acids, sex hormones, corticoids, cardenolides, cardenolide glycosides, bufadienolides, steroid sapogenins and steroid alkaloids.

2. Process as claimed in claim 1,
**characterized in that**
the hapten is selected from the group comprising cardenolides and cardenolide glycosides.

3. Process as claimed in claim 2,
**characterized in that**
the hapten is selected from the group comprising digoxigenin, digitoxigenin, gitoxigenin, strophanthidin, digoxin, digitoxin, ditoxin and strophanthin.

4. Process as claimed in claim 3,
**characterized in that**
digoxigenin or digoxin is used as the hapten.

5. Process as claimed in one of the claims 1 to 4,
**characterized in that**
an amino acid derivative having a first protective group for the amino side chain that can be quantitatively cleaved off under certain reaction conditions is used at positions of the peptide at which the hapten is to be coupled, and an amino acid derivative having a second protective group for the amino side chain which itself is not cleavable under the reaction conditions that cleave the first protective group is used at positions of the peptide where the hapten is not to be coupled.

6. Process as claimed in claim 5,
**characterized in that**
a first acid-labile protective group and a second acid-stable protective group is used.

7. Process as claimed in one of the claims 1 to 6,
**characterized in that**
an N-hydroxy succinimide ester is used as the active ester derivative.

8. Process as claimed in one of the claims 1 to 7,
**characterized in that**
a peptide is synthesized comprising an immunologically reactive epitope region and a spacer region, at least one hapten label being coupled to the spacer region.

9. Process as claimed in claim 8,
**characterized in that**
the spacer region has a length of 1 to 10 amino acids.

10. Process as claimed in claim 8 or 9,
**characterized in that**
a spacer region is located at the amino or/and carboxy terminus of the peptide.

11. Process as claimed in one of the claims 8 to 10,
**characterized in that**
the spacer region contains amino acids which have charges or/and can form hydrogen bridges.

12. Process as claimed in one of the claims 8 to 11,
**characterized in that**
the amino acids of the spacer region are selected from the group comprising glycine, β-alanine, γ-aminobutyric acid, ∈-aminocaproic acid, lysine and compounds of the structural formula NH₂-[(CH₂)ₙ-O]ₓ-CH₂-CH₂-COOH, in which n is 2 or 3 and x is 1 to 10.

13. Process as claimed in one of the claims 1 to 12,
**characterized in that**
a peptide is synthesized which contains an epitope region derived from the amino acid sequences of HIV I, HIV II or HCV.

14. Process as claimed in claim 13,
**characterized in that**
the epitope region is selected from the group of HIV I or HIV II amino acid sequences 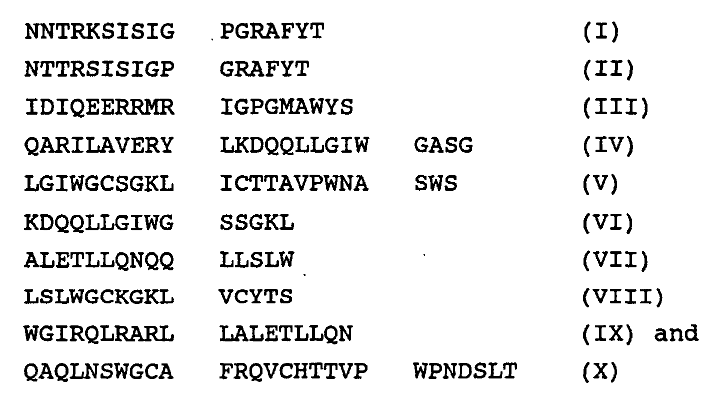 or partial sequences thereof which have a length of at least 6 amino acids.

15. Process as claimed in claim 13,
**characterized in that**
the epitope region is selected from the group of HCV amino acid sequences 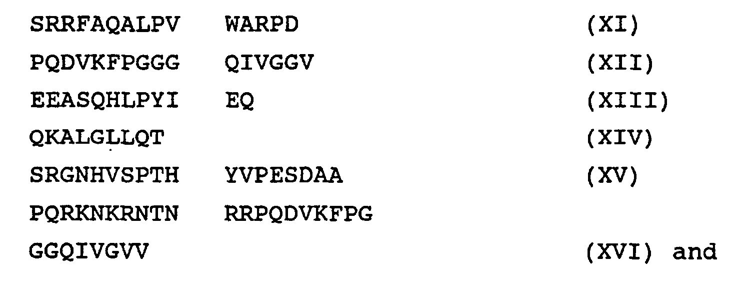 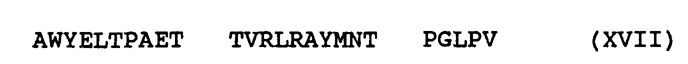 or partial sequences thereof which have a length of at least 6 amino acids.

16. Hapten-labelled peptide which has a maximum length of 50 amino acids and is coupled at the amino terminus or/and at amino side groups with at least one hapten-active ester derivative, wherein the hapten is selected from the group comprising sterols, gallic acids, sex hormones, corticoids, cardenolides, cardenolide glycosides, bufadienolides, steroid sapogenins and steroid alkaloids, and wherein
the peptide comprises an immunologically reactive epitope region and an immunologically non-reactive spacer region the spacer region carrying at least one hapten label.

17. Peptide as claimed in claim 16,
**characterized in that**
the hapten is digoxigenin or digoxin.

18. Peptide as claimed in claim 16,
**characterized in that**
a spacer region is located at the amino or/and carboxy terminus of the peptide.

19. Peptide as claimed in one of the claims 16 to 18,
**characterized in that**
the epitope region is derived from the amino acid sequences of HIV I, HIV II or HCV.

20. Peptide as claimed in claim 19,
**characterized in that**
the epitope region is selected from the group of HIV I or HIV II amino acid sequences 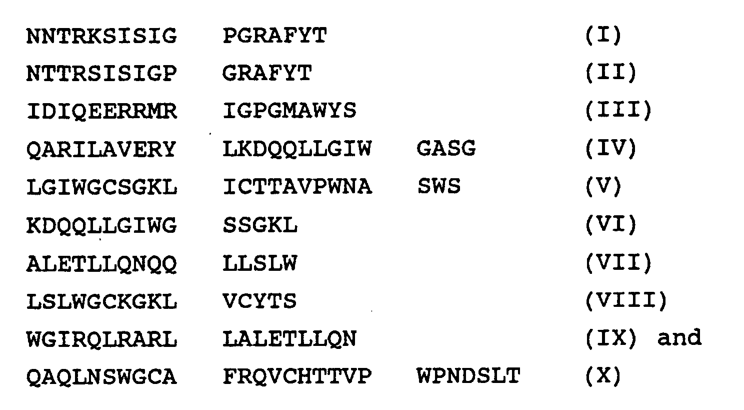 or partial sequences thereof which have a length of at least 6 amino acids.

21. Peptide as claimed in claim 19,
**characterized in that**
the epitope region is selected from the group of HCV amino acid sequences 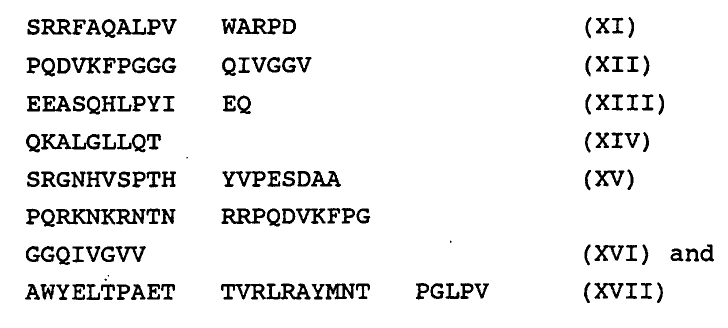 or partial sequences thereof which have a length of at least 6 amino acids.

22. Use of hapten-labelled peptides which have been produced by a process as claimed in one of the claims 1 to 15, or of peptides as claimed in one of the claims 16 to 21 as antigens in an immunological method for the determination of specific antibodies in a sample liquid.

23. Use as claimed in claim 22 in an immunological method in the bridge test format.

24. Method for the immunological determination of a specific antibody in a sample liquid,
**characterized in that**
the sample liquid is incubated with
(a) a first labelled antigen which is directed against the antibody to be determined and comprises a hapten-labelled peptide that has been produced by a process as claimed in one of the claims 1 to 15, or a peptide as claimed in one of the claims 16 to 21 and
(b) a receptor for the hapten which carries a signal-generating group,
and binding to the peptide is used to detect the antibody.

25. Method as claimed in claim 24,
**characterized in that**
a peptide labelled with digoxin or digoxigenin is used as the first antigen and an antibody directed against digoxigenin or/and digoxin is used as the receptor.

26. Method as claimed in claim 24 or 25,
**characterized in that**
the sample liquid is incubated in the presence of a solid phase with the first antigen and with a second antigen that is directed against the antibody to be determined and
(a) is bound to the solid phase or
(b) is present in a form that can be bound to the solid phase,
and the antibody to be determined is detected by determining the label in the solid phase or/and in the liquid phase.

27. Method as claimed in claim 26,
**characterized in that**
an antigen labelled with biotin is used as the second antigen and a solid phase is used which is coated with streptavidin or avidin.

28. Method as claimed in claim 27,
**characterized in that**
a peptide labelled with biotin is used as the second antigen.

29. Method as claimed in one of the claims 26 to 28,
**characterized in that**
the sample liquid is mixed with the first and the second antigen and subsequently the receptor for the hapten of the first antigen is added.

30. Reagent for the immunological determination of a specific antibody,
**characterized in that**
it contains at least one hapten-labelled peptide which reacts with the antibody to be determined and has been produced by a process as claimed in one of the claims 1 to 15, or a hapten-labelled peptide as claimed in one of the claims 16 to 21 which reacts with the antibody to be determined.

31. Reagent as claimed in claim 30,
**characterized in that**
it contains
(a) the hapten-labelled peptide which reacts with the antibody to be determined,
(b) a receptor for the hapten which carries a signal-generating group and
(c) an additional antigen which reacts with the antibody to be determined and is bound to a solid phase or is present in a form that is capable of binding to a solid phase.

## Revendications

1. Procédé de préparation de peptides marqués par des haptènes, **caractérisé en ce que**
(a) on synthétise un peptide ayant la séquence d'aminoacides désirée sur une phase solide à partir de dérivés d'aminoacides dont les groupes latéraux réactifs sont bloqués par des groupes protecteurs, les groupes protecteurs de groupes latéraux amino primaire étant choisis de façon qu'ils puissent éventuellement se séparer sélectivement,
(b) on sépare des groupes protecteurs en formant au moins un groupe amino libre,
(c) on couple un dérivé ester actif d'un haptène à au moins un groupe amino primaire libre du peptide, et
(d) on sépare éventuellement les groupes protecteurs encore restants,
le haptène étant choisi dans le groupe constitué par des stérols, des acides biliaires, des hormones sexuelles, des corticoïdes, des cardénolides, des glucosides de cardénolide, des bufadiénolides, des sapogénines stéroïdiennes et des alcaloïdes stéroïdiens.

2. Procédé selon la revendication 1, **caractérisé en ce que** l'haptène est choisi dans le groupe constitué par des cardénolides et des glucosides de cardénolides.

3. Procédé selon la revendication 2, **caractérisé en ce que** l'haptène est choisi dans le groupe constitué par la digoxigénine, la digitoxigénine, la gitoxigénine, la strophantidine, la digoxine, la digitoxine, ;la ditoxine et la strophantine.

4. Procédé selon la revendication 3, **caractérisé en ce que** l'on utilise la digoxigénine ou la digoxine comme haptène.

5. Procédé selon l'une des revendications 1 à 4, **caractérisé en ce que**, aux positions du peptide auxquelles doit s'effectuer le couplage de l'haptène, on utilise un dérivé d'aminoacide ayant un premier groupe protecteur pour la chaîne latérale amino pouvant se séparer de manière quantitative dans certaines conditions réactionnelles, et **en ce que**, aux positions du peptide auxquelles il ne doit pas s'effectuer de couplage de l'haptène, on utilise un dérivé d'aminoacide ayant un deuxième groupe protecteur pour la chaîne latérale amino, qui ne se sépare pas dans les conditions réactionnelles dans lesquelles se sépare le premier groupe protecteur.

6. Procédé selon la revendication 5, **caractérisé en ce que** l'on utilise un premier groupe protecteur labile en milieu acide et un deuxième groupe protecteur stable en milieu acide.

7. Procédé selon l'une des revendications 1 à 6, **caractérisé en ce que** l'on utilise comme dérivé ester actif un ester de N-hydroxysuccinimide.

8. Procédé selon l'une des revendications 1 à 7, **caractérisé en ce que** l'on synthétise un peptide qui comprend un domaine épitopique immunologiquement réactif et un domaine espaceur, au moins un haptène marqueur étant couplé au domaine espaceur.

9. Procédé selon la revendication 8, **caractérisé en ce que** le domaine espaceur a une longueur de 1 à 10 aminoacides.

10. Procédé selon la revendication 8 ou 9, **caractérisé en ce qu'**un domaine espaceur est situé à l'extrémité amino et/ou carboxy du peptide.

11. Procédé selon l'une des revendications 8 à 10, **caractérisé en ce que** le domaine espaceur contient des aminoacides qui sont chargés et/ou peuvent former des liaisons hydrogène.

12. Procédé selon l'une des revendications 8 à 11, **caractérisé en ce que** les aminoacides du domaine espaceur sont choisis dans le groupe constitué par la glycine, la β-alanine, l'acide γ-aminobutyrique, l'acide ε-aminocaproïque, la lysine et les composés de formule structurale NH₂-[(CH₂)ₙ-O]ₓ-CH₂-CH₂-COOH, dans laquelle n est 2 ou 3 et x est compris entre 1 et 10.

13. Procédé selon l'une des revendications 1 à 12, **caractérisé en ce que** l'on synthétise un peptide contenant un domaine épitopique issu des séquences d'aminoacides de VIH I, VIH II ou VHC.

14. Procédé selon la revendication 13, **caractérisé en ce que** le domaine épitopique est choisi dans le groupe des séquences d'aminoacides de VIH I ou VIH II 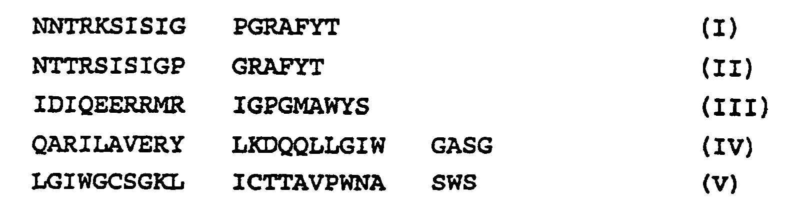 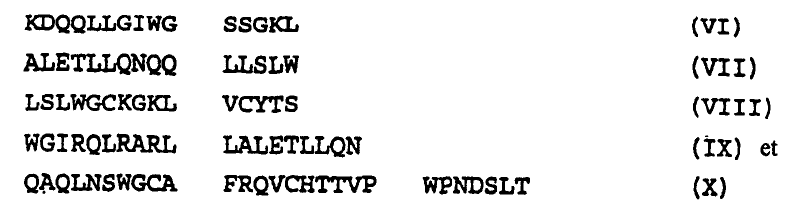 ou des parties de ces séquences ayant une longueur d'au moins 6 aminoacides.

15. Procédé selon la revendication 13, **caractérisé en ce que** le domaine épitopique est choisi dans le groupe des séquences d'aminoacides de VHC 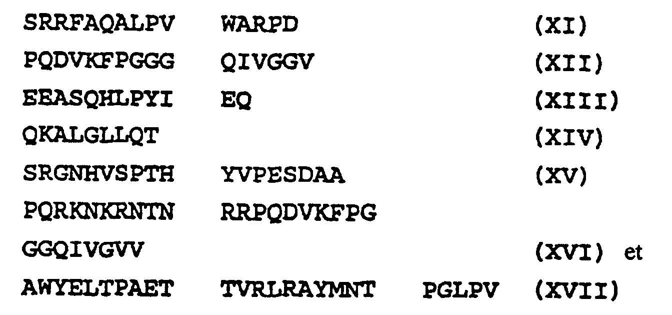 ou des parties de ces séquences ayant une longueur d'au moins 6 aminoacides.

16. Peptide marqué par un haptène, qui a une longueur d'au plus 50 aminoacides et qui est couplé à l'extrémité amino ou au niveau de groupes latéraux amino avec au moins un dérivé ester actif d'un haptène, l'haptène étant choisi dans le groupe constitué par des stérols, des acides biliaires, des hormones sexuelles, des corticoïdes, des cardénolides, des glucosides de cardénolide, des bufadiénolides, des sapogénines stéroïdiennes et des alcaloïdes stéroïdiens, et le peptide comprenant un domaine épitopique immunologiquement réactif et un domaine espaceur non immunologiquement réactif, le domaine espaceur portant au moins un haptène marqueur.

17. Peptide selon la revendication 16, **caractérisé en ce que** l'haptène est la digoxigénine ou la digoxine.

18. Peptide selon la revendication 16, **caractérisé en ce qu'**un domaine espaceur est situé à l'extrémité amino et/ou carboxy du peptide.

19. Peptide selon l'une des revendications 16 à 18, **caractérisé en ce que** le domaine épitopique est issu des séquences d'aminoacides de VIH I, de VIH II ou de VHC.

20. Peptide selon la revendication 19, **caractérisé en ce que** le domaine épitopique est choisi dans le groupe des séquences d'aminoacides de VIH I ou VIH II 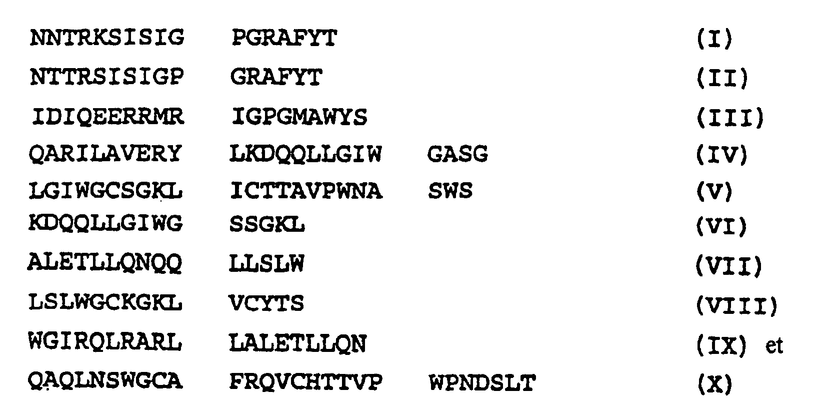 ou des parties de ces séquences ayant une longueur d'au moins 6 aminoacides.

21. Peptide selon la revendication 19, **caractérisé en ce que** le domaine épitopique est choisi dans le groupe des séquences d'aminoacides de VHC 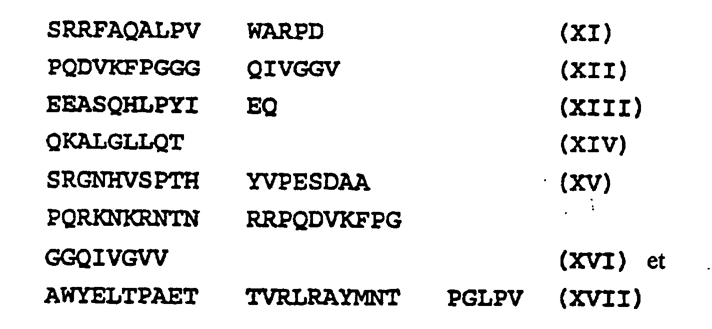 ou des parties de ces séquences ayant une longueur d'au moins 6 aminoacides.

22. Utilisation de peptides marqués par des haptènes préparés par un procédé selon l'une des revendications 1 à 15, ou de peptides selon l'une des revendications 16 à 21, comme antigènes dans un procédé immunologique de dosage d'anticorps spécifiques dans un échantillon liquide.

23. Utilisation selon la revendication 22 dans un procédé immunologique dans un format d'analyse en pont.

24. Procédé de dosage immunologique d'un anticorps spécifique dans un échantillon liquide, **caractérisé en ce que** l'on met l'échantillon liquide à incuber avec
(a) un premier antigène marqué, dirigé contre l'anticorps à doser, et comprenant un peptide marqué par un haptène préparé par un procédé selon l'une des revendications 1 à 15 ou un peptide selon l'une des revendications 16 à 21, et
(b) un récepteur pour l'haptène, portant un groupe générateur d'un signal,
et on décèle l'anticorps par l'intermédiaire d'une liaison avec le peptide.

25. Procédé selon la revendication 24, **caractérisé en ce que** l'on utilise comme premier antigène un peptide marqué avec la digoxine ou la digoxigénine et comme récepteur un anticorps dirigé contre la digoxigénine et/ou la digoxine.

26. Procédé selon la revendication 24 ou 25, **caractérisé en ce que** l'on met l'échantillon liquide à incuber en présence d'une phase solide avec le premier antigène et un deuxième antigène qui est dirigé contre l'anticorps à déterminer et qui
(a) est lié à la phase solide ou
(b) se présente sous une forme pouvant se lier à la phase solide,
et on décèle l'anticorps à déterminer en dosant le marqueur dans la phase solide et/ou dans la phase liquide.

27. Procédé selon la revendication 26, **caractérisé en ce que** l'on utilise comme deuxième antigène un antigène marqué avec de la biotine, et une phase solide revêtue de streptavidine ou d'avidine.

28. Procédé selon la revendication 27, **caractérisé en ce que** l'on utilise comme deuxième antigène un peptide marqué avec de la biotine.

29. Procédé selon l'une des revendications 26 à 28, **caractérisé en ce que** l'on mélange l'échantillon liquide avec le premier et le deuxième antigène, puis on ajoute le récepteur pour l'haptène du premier antigène.

30. Réactif pour le dosage immunologique d'un anticorps spécifique, **caractérisé en ce qu'**il contient au moins un peptide marqué par un haptène et réagissant avec l'anticorps à déterminer, préparé par un procédé selon l'une des revendications 1 à 15, ou un peptide marqué par un haptène et réagissant avec l'anticorps à déterminer selon l'une des revendications 16 à 21.

31. Réactif selon la revendication 30, **caractérisé en ce qu'**il contient
(a) le peptide marqué par un haptène, réagissant avec l'anticorps à déterminer,
(b) un récepteur pour l'haptène, qui porte un groupe générateur de signal, et
(c) un autre antigène réagissant avec l'anticorps à déterminer, qui est lié à une phase solide ou qui se trouve sous une forme pouvant se lier à une phase solide.
